# EUROPEAN PATENT APPLICATION

(11) **EP 4 066 843 A1**
(43) Date of publication of application: **05.10.2022**
(21) Application number: 21190154.1
(22) Date of filing: 06.08.2021
(51) Int. Cl.: A61K 35/644, A61K 33/38, A61K 33/40, A61K 36/82, A61P 17/02

(54) **WOUND HONEY**

(30) Priority: 31.03.2021 US 202163207937 P
(71) Applicant: Fenzl, Mark Edward, Sarasota, FL 34232 (US)
(72) Inventor: Fenzl, Mark Edward, Sarasota, FL 34232 (US)
(74) Representative: Sach, Greg Robert

(57) **Abstract**

A local honey mixed with a natural preservative concentrate to create a more cost effective medical product. This local product may have the advantageous effect of reducing seasonal allergies and asthma. The product will have the antimicrobial synergistic advantage of combining cinnamaldehyde with vanillin; the synergistic bactericidal process of high pressure processing of a product containing cinnamaldehyde; the synergistic antibacterial effect of combining hydrogen peroxide with organic acids; and the synergistic bactericidal effect of combining cinnamaldehyde with silver nanoparticles. This product will be cheaper than high MGO manuka honey. Additionally, the wound or burn product may also be used as a diagnostic ultrasound gel or sanitizing gel if necessary.

## Description

### CROSS-REFERENCE TO RELATED APPLICATIONS

This application claims the benefit of U.S. Provisional Application 63/207,937, filed on March 31, 2021. The entire disclosure of the above application is incorporated herein by reference.

### FIELD

The present disclosure relates to a medical product and, more particularly, to a product containing honey with natural antimicrobials that has been discovered.

### BACKGROUND

This section provides background information related to the present disclosure which is not necessarily prior art.

The present invention relates to an improved wound product that is equivocal to manuka honey in antimicrobial and wound healing effectiveness, but is significantly cheaper. Manuka Honey comes from Australia and New Zealand because that is where *Leptospermum scoparium* trees grow. Manuka honey has been studied for its antimicrobial and wound healing properties and honey containing an appropriate amount of methylglyoxal has been approved to be used as a wound honey. The problem with Manuka Honey is that it is limited in location to these areas of the world, there is a limit to the production of Manuka Honey, and it is very expensive. Because of cost, many people can not afford Manuka Honey. Regular honey contains hydrogen peroxide instead of methylglyoxal, but by substituting natural antimicrobial products for methylglyoxal, one can create a similar, local product at a lower cost.

A benefit of using local honey is that it is less likely to cause allergic reactions and will benefit people with seasonal allergies and asthma. People in a local area have already been exposed to the local pollen, decreasing the risk of sensitivity response and adverse reaction. People with allergies are often treated with minute doses of allergens to build up tolerance. By using local honey, people that have seasonal allergies that use the wound honey may have an improvement in their seasonal allergies and / or asthma.

Two ways a product is protected from microbial spoilage are water activity level and acidity. Products with low water activity level do not have enough water for bacteria or fungus reproduce and cause damage. Acidity or low pH damage microbes, preventing them from damaging the product. Honey naturally has a low water activity level and acidity. By adding ingredients with low water activity and acidity, such as a non-limiting example of citric acid, further protects the product.

The only microbes found in honey are found in spore form. Examples of this include *Clostridium botulinum* which may cause botulinum poisoning and *Bacillus subtilis,* which may cause septicemia in immunocompromised persons. Current wound honey uses gamma irradiation to destroy these spores and sterilize the honey. There are natural products that are known to inhibit and kill these spores. Non-limiting examples include a variety of natural products. Organic acids, such as acetic acid or parecetic acid, inhibit bacterial spores. Aldehydes, such as benzaldehyde found in bitter almond essential oil, cinnamaldehyde found in cinnamon essential oil, citral found in lemongrass essential oil, and vanillin found in vanilla inhibit vegetative cells and spore revival. Fatty acid esters, such as monolaurin or caprylin, inhibit bacterial spores. Green tea polyphenols, such as epigallocatechin gallate (EGCG), have demonstrated the ability to irradicate bacterial spores. Metal nanoparticles, such as silver nanoparticles, inhibit bacterial spores and silver nanoparticles have a synergistic sporicidal effect with cinnamaldehyde. Organic acids, aldehydes, fatty acid esters, green tea polyphenols, and metal nanoparticles have broad spectrum antimicrobial properties. High pressure processing is a means of putting a product under pressure for a period of time in order to damage bacteria and bacterial spores. This is a means to protect juices from bacterial contamination without the damage of heating or pasteurization. These natural antimicrobials in combination with high pressure processing can provide sterilization without the problems of shipping to and from a radiation facility, time lost for shipping to a radiation facility, and cost of radiation sterilization.

High pressure processing is a way to destroy bacteria and inactivate bacterial spores without heating. There is a synergistic effect of using cinnamaldehyde and high pressure processing to kill resistant bacteria and bacterial spores. Please note table 1.

**Table 1. Mean logarithmic decrease in spores of B. cereus over 6 weeks after treatment with high pressure at 600 MPA (HP), application of 0.1% trans-cinnamaldehyde (TC), or both (HP + TC) at 7° Celcius in infant formula.**

| Treatment | Week 1 | Week 2 | Week 3 | Week 4 | Week 5 | Week 6 |
|---|---|---|---|---|---|---|
| Control | 0.5 | 0.5 | 1 | 1 | 1 | 1 |
| HP | 2 | 2.25 | 2.5 | 3 | 3.25 | 3.25 |
| TC | 1.5 | 2 | 3 | 2.25 | 3 | 1.5 |
| HP + TC | 3.25 | 3.75 | 4 | 3.5 | 4.25 | 4 |

Honey is naturally preserved with hydrogen peroxide, polyphenols, and organic acids. Although this combination will prevent bacteria from growing in honey, it may not necessarily be bactericidal as a wound dressing. Some honeys with high levels of hydrogen peroxide, such as honeydew honey, are bactericidal at lower concentrations than manuks honey. There is a synergistic bactericidal effect between organic acids and hydrogen peroxide. These may be added to honey for a bactericidal effect. Please see Table 2.

**Table 2. Synergistic bactericidal combination of hydrogen peroxide and organic acids in percentage.**

| Acid | Bacterial Strain | MBC acid | MBC hydrogen peroxide | MBC combination Acid / H2O2 In Bovine albumin + yeast extract 1% |
|---|---|---|---|---|
| Formic | *Enterococcus hirae* | 5% | 12.5% | 0.625 % / 3.12 % |
| Formic | *Staphylococcus aureus* | 1.25% | 3.12% | 0.312 % / 1.56% |
| Formic | *Listeria monocytogenes* | 1.56% | 6.25% | 0.625 % / 0.78% |
| Formic | *Pseudomonas aeruginosa* | 0.025% | 1.56% | 0.025 % / 0.39% |
| Acetic | *Pseudomonas aeruginosa* | 0.625% | 1.56% | 0.625 % / 0.39% |
| Succinic | *Pseudomonas aeruginosa* | 0.078% | 1.56% | 0.078 % / 0.78% |
| Propionic | *Pseudomonas aeruginosa* | 0.5% | 1.56% | 0.50% / 0.19% |

One example of several potential products that does not limit this patent application, is local honey admixed with a natural preservative concentrate comprising between 250 and 250,000 parts per million vanillin, between 50 and 50,000 parts per million hydrogen peroxide, between 50 and 50,000 parts per million cassia essential oil, and between 10 and 10,000 parts per million silver nanoparticles. The honey is mixed and put through high pressure processing to kill any residual spores. There is antibacterial synergy between cinnamaldehyde and vanillin. There is a synergistic effect of cinnamaldehyde and silver nanpoarticles as seen in Table 3.

**Table 3. Minimal Inhibitory Concentration of silver nanoparticles and cinnamaldehyde and Fractional Inhibitory Concentration Index (FICI).**

| Bacteria | Silver nanoparticles (ng/ml) | Cinnamaldehyde (mg/ml) | Synergy Silver nanparticles (ng/ml) | Synergy Cinnamadehyde (mg/ml) | FICI |
|---|---|---|---|---|---|
| Clostridium perfringens | 612.5 | 0.065 | 153.1 | 0.016 | 0.5 |
| Bacillus cereus | 1,225.0 | 0.328 | 306.3 | 0.082 | 0.5 |

A second example would be sugarcane molasses mixed with a natural preservative concentrate comprising between, between 250 and 250,000 parts per million formic acid, between 50 and 50,000 parts per million hydrogen peroxide, and between 50 and 50,000 parts per million cassia essential oil. The molasses is mixed and put through high pressure processing. Sugarcane molasses has an antimicrobial effect against multiple bacteria as seen in Table 4.

**Table 4. Minimal Inhibitory Concentration and Minimal Bactericidal Concentration of sugarcane molasses extract**

| Microorganism | MIC | **MBC** |
|---|---|---|
| *S. aureus ATCC29737* | 3.12% | 6.25% |
| *L. monocytogenes ATCC 19112* | 3.13% | 6.25% |
| *E. coli* ATCC 10536 | 6.25% | 12.50% |
| *S. enteric* ser. Typhimurium | 6.25% | >12.5% |

A third example would be Manuka Honey mixed with a natural preservative concentrate comprising between 250 and 250,000 parts per million acetic acid, and between 50 and 50,000 parts per million lemongrass essential oil. The manuka honey is mixed and put through high pressure processing before sale. This product may have a pleasant lemon scent.

Using an essential oil provides a pleasing scent, which creates an enjoyable experience for the patient and health care provider. It may also cover any unpleasant odors from the wound. Mixing and selling the product locally with local honey or honey substitute will significantly decrease shipping and 3^{rd} party costs for the final product. This will allow people that can not afford expensive manuka honey an opportunity to use a similar wound or burn product to heal their wounds.

Advantageously, this product may also be used as an ultrasound gel or a surgical sanitizer. This wound gel or burn gel may be placed on a wound site. If the patient needs diagnostic imaging, an ultrasound probe may be placed in the wound gel or burn gel and provide diagnostic information without having to clean the wound product off to place expensive sterile ultrasound gel. Or if the patient is scheduled for surgery, the wound gel or burn gel would not need to be removed prior to surgery as it is acting as the surgical sanitizer.

### SUMMARY OF THE INVENTION

In concordance with the instant disclosure, this invention is an improved natural honey or honey substitute product that can be used as a medical product with the addition of natural preservatives. With the addition of natural preservatives, this product may be equivalent in antimicrobial and wound healing efficacy to Manuka honey, but may take less time for transport and cost less for materials. This product may benefit people that have allergic reactions to pollen, those with seasonal allergies, and those with asthma. One example of several potential medical products that does not limit this patent application, is local honey admixed with a natural green tea extract catechins, an aldehyde, fatty acid esters, and metal nanoparticles. The product honey is put through high pressure processing to kill any persistant spores. It can then be used as a local wound honey. Another example may be molasses mixed with an organic acid, hydrogen peroxide, and a monoterpene. A third example may be Manuka honey containing an organc acid, hydrogen peroxide, and an essential oil.

It may contain a natural essential oil that can create a pleasant scent and hide odor. There would also be significant decrease in cost due to decreased cost of shipping and storage and the use of local honey instead of expensive imported Manuka Honey. There would be no lost time, expense, or transportation for gamma irradiation because bacterial spores would be destroyed through natural preservatives and high pressure processing. A dual use wound gel / burn gel that is also an ultrasound gel / surgical sanitizer will save time and money in the hospital, burn center, or wound center.

### DETAILED DESCRIPTION

The following description of technology is merely exemplary in nature of the subject matter, manufacture and use of one or more inventions, and is not intended to limit the scope, application, or uses of any specific invention claimed in this application or in such other applications as may be filed claiming priority to this application, or patents issuing therefrom. Regarding methods disclosed, the order of the steps presented is exemplary in nature, and thus, the order of the steps can be different in various embodiments, including where certain steps can be simultaneously performed. "A" and "an" as used herein indicate "at least one" of the item is present; a plurality of such items may be present, when possible. Except where otherwise expressly indicated, all numerical quantities in this description are to be understood as modified by the word "about" and all geometric and spatial descriptors are to be understood as modified by the word "substantially" in describing the broadest scope of the technology. "About" when applied to numerical values indicates that the calculation or the measurement allows some slight imprecision in the value (with some approach to exactness in the value; approximately or reasonably close to the value; nearly). If, for some reason, the imprecision provided by "about" and/or "substantially" is not otherwise understood in the art with this ordinary meaning, then "about" and/or "substantially" as used herein indicates at least variations that may arise from ordinary methods of measuring or using such parameters.

Although the open-ended term "comprising," as a synonym of non-restrictive terms such as including, containing, or having, is used herein to describe and claim embodiments of the present technology, embodiments may alternatively be described using more limiting terms such as "consisting of' or "consisting essentially of." Thus, for any given embodiment reciting materials, components, or process steps, the present technology also specifically includes embodiments consisting of, or consisting essentially of, such materials, components, or process steps excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein.

Disclosures of ranges are, unless specified otherwise, inclusive of endpoints and include all distinct values and further divided ranges within the entire range. Thus, for example, a range of "from A to B" or "from about A to about B" is inclusive of A and of B. Disclosure of values and ranges of values for specific parameters (such as amounts, weight percentages, etc.) are not exclusive of other values and ranges of values useful herein. It is envisioned that two or more specific exemplified values for a given parameter may define endpoints for a range of values that may be claimed for the parameter. For example, if Parameter X is exemplified herein to have value A and also exemplified to have value Z, it is envisioned that Parameter X may have a range of values from about A to about Z. Similarly, it is envisioned that disclosure of two or more ranges of values for a parameter (whether such ranges are nested, overlapping or distinct) subsume all possible combination of ranges for the value that might be claimed using endpoints of the disclosed ranges. For example, if Parameter X is exemplified herein to have values in the range of 1-10, or 2-9, or 3-8, it is also envisioned that Parameter X may have other ranges of values including 1-9, 1-8, 1-3, 1-2, 2-10, 2-8, 2-3, 3-10, 3-9, and so on.

The present disclosure relates to a natural sporicidal antimicrobial that is mixed in with a honey or honey substitute, as described in greater detail herein below.

One example of several potential medical products that does not limit this patent application, is local honey admixed with a natural preservative concentrate comprising 2,000 parts per million green tea with 50% catechins (0.2%), 1,400 parts per million vanillin (0.14%), 1,000 parts per million cassia essential oil (0.1%) and 1,000 parts per million silver nanoparticles (0.1%). The honey is mixed and put through high pressure processing in a local facility that mixes the concentrate with local honey, packages the final product, and sells the final product. Vanillin and cinnamaldehyde have a synergistic antimicrobial effect. Hydrogen peroxide has a synergistic antibacterial effect with organic acids found in honey. Cinnamaldehyde and high pressure processing have a synergistic bactericidal effect. Essential oils with monoterpines and organic acids have a synergistic sporicidal effect. It could be used as an over-the-counter wound honey.

A second example would be sugarcane molasses mixed with a natural preservative concentrate comprising 3,000 parts per million formic acid (0.3%), 15,000 parts per million hydrogen peroxide (1.5%), and 1,000 parts per million cassia essential oil (0.1%). The molasses is mixed and put through high pressure processing. It could be used as a wound or burn product.

A third example would be a Manuka Honey mixed with a natural preservative concentrate comprising 1,000 parts per million formic acid (0.1%), 15,000 parts per million hydrogen peroxide (1.5%), and 1,000 parts per million lemongrass essential oil (0.1 %). The manuka honey is mixed and put through high pressure processing. This product may have a pleasant lemon scent. It can be used as a combination wound honey and surgical sanitizer.

It is desirable for the supplement to have a low water activity level. Honey naturally has a low water activity level containing approximately 17% water. Other additional humectants may include nitric acid, dextrose, fructose, glycerol, glycine, glucose, malic acid, salt, sorbitol, sucrose, and tartaric acid. A skilled artisan may select any suitable humectant, as desired. For example, the supplement can have a water activity level of 0.86.

It is also desirable for a product to have a low pH as this inhibits microbes. Honey typically has a pH between 3.5 and 5.5. Acids lower the pH. Some acids, such as formic acid, acetic acid, oxalic acid, nitric acid, lactic acid, succinic acid, tartaric acid, citric acid, propionic acid, sulfhuric acid, benzoic acid, sorbic acid, sulphamic acid, phosphoric acid, mandelic acid, and adipic acid have synergistic antimicrobial effects with hydrogen peroxide against bacteria. Hydrogen peroxide is naturally found in non-mauka type honeys.

Additionally, as may be presented in the claims herein, the language "consisting of' is intended to exclude any ingredient not specified in the claim. Accordingly, the supplement consisting of certain ingredients described hereinabove includes only those ingredients.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms, and that neither should be construed to limit the scope of the disclosure. In some example embodiments, well-known processes, well-known device structures, and well-known technologies are not described in detail. Equivalent changes, modifications and variations of some embodiments, materials, compositions and methods can be made within the scope of the present technology, with substantially similar results.

## Claims

1. A wound or burn product comprising at least two antimicrobials that are admixed with at least one of honey, artificial honey, molasses, nectar, and syrup.

2. A product in claim #1 where at least one of the antimicrobials is a catechin.

3. A product in claim #1 where at least one of the antimicrobials is an aldehyde.

4. A product in claim #1 where at least one of the antimicrobials is a fatty acid ester.

5. A product in claim #1 where at least one of the antimicrobials is an essential oil monoterpine.

6. A product in claim #1 where at least one of the antimicrobials is an organic acid.

7. A product in claim #1 where at least one of the antimicrobials is a peroxide.

8. A product in claim #1 where at least one of the antimicrobials is a metal particle.

9. The process of high pressure processing to destroy bacteria in a honey product.

10. A product in claim #9 where the product is an over-the-counter wound honey.

11. A product in claim #9 where the product is a burn product.

12. A product in claim #9 where the product is a wound product.

13. A product in claim #9 where the product is a surgical sanitizer.

14. A product in claim #9 where the product is food.

15. A natural preservative concentrate mixed with local honey to create a medical product.
